# FASCICULE DE BREVET EUROPEEN

(11) **EP 1 427 367 B1**
(45) Date de publication et mention de la délivrance du brevet: **31.10.2007**
(21) Numéro de dépôt: 02796294.3
(22) Date de dépôt: 14.08.2002
(51) Int. Cl.: A61F 9/00

(54) **BOUCHONS LACRYMAUX ET METHODES DE MISE EN PLACE DE CES DISPOSITIFS**
TRÄNENGANGSTOPFEN UND VERFAHREN ZU IHRER ANBRINGUNG
LACHRYMAL PLUGS AND METHODS FOR SETTING SAME

(30) Priorité: 31.08.2001 FR 0111324
(43) Date de publication de la demande: 16.06.2004
(73) Titulaire: Fouere, Alain, 13008 Marseille (FR); Bige, Pierre, 83270 Saint-Cyr sur Mer (FR)
(72) Inventeur: Fouere, Alain, 13008 Marseille (FR); Bige, Pierre, 83270 Saint-Cyr sur Mer (FR)
(74) Mandataire: Roman, Michel
(86) Numéro de dépôt international: PCT/FR2002/002878
(87) Numéro de publication internationale: WO 2003/017897

(56) Documents cités:
- US-A- 5 830 171
- US-B1- 6 254 562

## Description

La présente invention a pour objet des bouchons lacrymaux, ainsi que des méthodes de mise en place de ces dispositifs.

Elle se rapporte d'une manière générale aux moyens destinés à contrôler le flux de larmes s'écoulant de la surface de l'oeil vers les fosses nasales.

Le fonctionnement normal d'un oeil humain nécessite que sa surface externe soit recouverte en permanence d'un film lubrifiant constitué par les larmes qui assurent à la fois un rinçage et une protection grâce aux antibiotiques naturels anti-infectieux qu'elles contiennent. Elles sont produites par une série de glandes se trouvant dans les paupières et dans le pourtour de l'oeil.

La déficience du maintien de la stabilité de ce film sur le globe oculaire peut entraîner divers désagréments tels que picotements, irritations, sensations de brûlure et dégradation de la vision en cas de manque d'humidité de la surface de l'oeil.

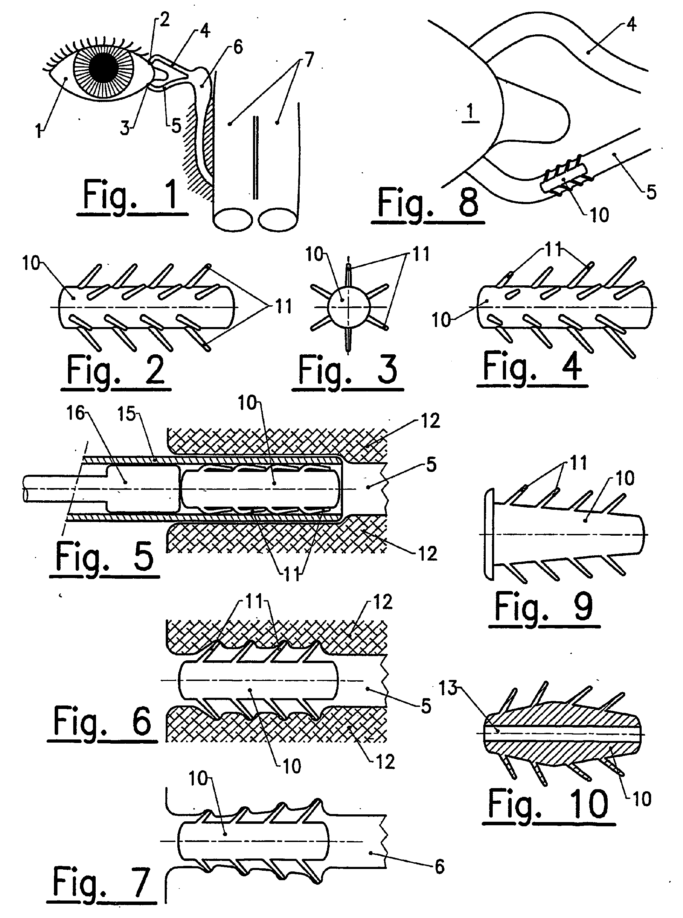

Les larmes sont produites continuellement, l'excès de fluide étant drainé de la surface du globe oculaire 1 à travers deux ouvertures ponctuelles constituant les points lacrymaux supérieur 2 et inférieur 3 situés près du coin interne de l'oeil et communiquant avec des conduits connus sous le nom de canalicules 4, 5 aboutissant à un sac lacrymal 6 qui se déverse dans la fosse nasale 7. Les points lacrymaux 2, 3 ont la faculté de s'ouvrir ou de se fermer à la manière d'un muscle de type sphincter, de façon à assurer la régulation du débit de fluide (figure 1).

Le manque de fluide lacrymal sur le globe oculaire est dû en général à une insuffisance des glandes productrice pouvant être provoqué par l'âge ou par d'autres causes.

Il est possible d'améliorer la situation en agissant sur les conduits lacrymaux, en les bouchant totalement ou partiellement.

En particulier, l'occlusion permanente, éventuellement par voie chirurgicale (cautérisation, laser), des conduits lacrymaux peut être une méthode de traitement des déficiences relatives aux larmes. Lorsque l'écoulement des larmes dans le sac naso-lacrymal est ainsi empêché, le volume des larmes restantes procure une plus forte humidité.

Cette méthode présente l'inconvénient d'être irréversible, à moins d'une nouvelle intervention chirurgicale. Pour y remédier, il a été proposé des dispositifs amovibles pouvant être mis en place dans un canal lacrymal et retiré sans intervention chirurgicale. Par exemple, le brevet US 334 137 déposé par la société "EAGLE VISION" décrit un dispositif de contrôle du fluide lacrymal bloquant le débit de ce fluide provenant de la surface de l'oeil et comportant une extrémité en tronc de cône inversé et une tête pourvue d'un dôme élargi. L'extrémité étant agencée pour faciliter le placement du dispositif à travers une ouverture ponctuelle et le dôme élargi empêchant la pénétration totale du dispositif dans la partie verticale du canalicule à travers l'ouverture ponctuelle.

Ce type de dispositifs présente toutefois des risques de migration ou d'expulsion accidentelles. Il nécessite en outre une instrumentation particulière pour la mise en place et l'extraction et ne permet pas d'augmenter le débit du fluide lacrymal.

Le brevet N° WO 98/33 461 déposé par M. Alain FOUERÉ, co-déposant de la présente demande, décrit un tampon méatique vissable destiné à être implanté dans les points lacrymaux et constitué d'un corps sensiblement cylindrique dont la surface latérale comporte un filet hélicoïdal semblable à celui d'une vis permettant de le mettre en place ou de le retirer par vissage ou dévissage, le tampon méatique comportant ou non un conduit traversant axial permettant le passage d'un débit déterminé de fluide lacrymal.

Le brevet N° US 6,254,562 B1 déposé par M. Alain FOUERÉ, co-déposant de la présente demande, décrit un tampon méatique vissable destiné à être implanté dans les points lacrymaux et constitué d'un corps sensiblement cylindrique dont la surface latérale comporte un filet hélicoïdal semblable à celui d'une vis permettant de le mettre en place ou de le retirer par vissage ou dévissage, le tampon méatique comportant ou non un conduit traversant axial permettant le passage d'un débit déterminé de fluide lacrymal. Le tampon méatique comporte aussi des griffes pour empêcher le vissage ou dévissage.

Le brevet N° US 5 830 171 déposé par M. Raymond G. WALLACE fait état d'un bouchon disposé au point lacrymal pour bloquer le flux de liquide lacrymal de la surface d'un oeil, ce bouchon comportant une tige s'insérant dans le point lacrymal et pourvue d'une extrémité distale avec rebord en forme d'aile pouvant, dans une première position, se rabattre le long de la tige pour permettre l'insertion facile du de la tige dans le point lacrymal et, dans une deuxième position, s'étendre radialement à extérieur de la tige pour empêcher le déplacement involontaire du rebord distal du point lacrymal.

Ces deux derniers implants ne permettent d'intervenir que sur les points lacrymaux 2, 3 et non sur les autres parties du système d'écoulement des larmes telles que les canalicules 4, 5 (cf figure 1).

En raison des leur conception, les dimensions des systèmes connus doivent être adaptés à la morphologie de chaque patient, ce qui nécessite la réalisation d'une gamme plus ou moins importante de prothèses de chaque type entrainant une élévation des coûts de fabrication et de stockage.

Le dispositif selon la présente invention, qui est essentiellement destiné à permettre l'occlusion des voies lacrymales, a pour objectif de remédier à cet état de choses pour permettre de lutter contre l'affection souvent désignée par le terme "oeil sec" en remédiant à une déficience des glandes lacrymales par la diminution ou la suppression de l'écoulement des larmes vers les fosses nasales.

Ce dispositif, particulièrement facile à implanter, présente l'avantage de s'adapter aisément à des morphologies différentes des conduits lacrymaux, ce qui permet l'utilisation d'une taille unique pour tous les patients.

Il est constitué d'un bouchon lacrymal destiné à être inséré dans les canalicules lacrymaux, ce bouchon, éventuellement pourvu d'un conduit axial, comportant sur ses parois externes des éléments flexibles rabattables sur lesdites parois pour permettre l'insertion du bouchon dans le conduit lacrymal et aptes à se redresser une fois le bouchon lacrymal installé de façon à maintenir ce dernier en place.

Sur les dessins annexés, donnés à titre d'exemples non limitatifs de formes de réalisation conformes à la présente demande :
la figure 1 déjà citée, représente schématiquement un oeil avec les canaux lacrymaux et le sac lacrymal,
les figures 2 et 3 montrent un bouchon lacrymal selon l'invention vu respectivement de côté et en bout,
la figure 4 représente une variante du bouchon lacrymal des figures 2 et 3 pourvue de griffes à longueur croissante,
la figure 5 est une vue en coupe axiale montrant la mise en place d'un bouchon lacrymal au moyen d'un tube à poussoir,
la figure 6 montre dans les mêmes conditions le bouchon lacrymal une fois mise en place,
la figure 7 représente une variante pourvue de griffes à longueur croissante du bouchon lacrymal de la figure 6,
la figure 8 montre un bouchon lacrymal selon la figure 6 implantée dans les conduits lacrymaux,
et les figures 9 et 10 sont des vues en coupe axiale de deux exemples de forme possible du dispositif objet de l'invention.

Le dispositif, figures 2 à 10, est constitué d'un corps 10 sensiblement cylindrique sur les parois latérales extérieures duquel sont implantées des éléments flexibles constitués de griffes 11 radiales pouvant être appliquées contre ces parois et se redresser lorsqu'elle sont libérées.

Ces éléments flexibles sont déterminées pour présenter une élasticité suffisante pour s'enfonçer partiellement dans la paroi interne 12 du canalicule 4, 5 en se redressant, de manière à assurer fermement le maintien en place du bouchon lacrymal (figure 5).

Les griffes 11 pourront être disposées en hélice ou toute autre configuration. Leur longueur pourra être constante, croissante (figures 4 et 7), décroissante, ou variable. Elles seront avantageusement inclinées en direction des fosses nasales 7, de façon à ne pas pouvoir être déplacées par le péristaltisme naturel du canal lacrymal entraînant larmes et corps étrangers vers l'intérieur.

La conformation exacte du corps 10 peut être variable. Il aura par exemple la forme d'un cône (figure 9), d'un double cône (figure 10) ou encore d'un diabolo.

Le corps 10 sera éventuellement pourvu d'un conduit axial 13 permettant un passage réduit des larmes (figure 10).

Le dispositif décrit pourra avantageusement comporter au moins un élément tel que disque flexible agencé pour assurer son étanchéité.

Le bouchon lacrymal pourra être réalisé en tout matériau permettant le repliage des griffes 11, ou autres éléments flexibles, et leur redressement, qu'il s'agisse de métal ou de matière synthétique. Il pourra en particulier être fabriqué en métal à mémoire de forme, ce qui peut donner la possibilité de l'installer sans instrument.

Il pourra éventuellement comporter un repère radio-opaque visible aux rayons X pour faciliter le repérage au cours de la progression du bouchon lacrymal lors de sa mise en place.

Cette mise en place s'effectura par tout moyen approprié permettant de plaquer les griffes 11 contre la paroi externe du corps 10 et de les libérer une fois la prothèse en place.

Ce moyen pourra en particulier consister en un tube 15 à poussoir 16 (figure 5) ou ou par un instrument pourvu de machoires similaires à celles d'un porte-mine. Un tel instrument serait en outre parfaitement adapté pour effectuer le retrait de la prothèse.

Le positionnement des divers éléments constitutifs donne à l'objet de l'invention un maximum d'effets utiles qui n'avaient pas été, à ce jour, obtenus par des dispositifs similaires.

## Revendications

1. Bouchon lacrymal destiné essentiellement à permettre l'occlusion des voies lacrymales pour remédier à une déficience des glandes lacrymales par la diminution ou la suppression de l'écoulement des larmes vers les fosses nasales, constituée d'un corps (10) sensiblement cylindrique sur les parois latérales extérieures duquel sont implantés des éléments flexibles pouvant être appliquées contre les parois dudit corps cylindrique pour permettre l'insertion du bouchon lacrymal, et se redresser lorsqu'ils sont libérés de façon à maintenir ledit bouchon lacrymal en place, où le bouchon est agencé pour être implanté le long d'un conduit lacrymal (4, 5) et les éléments flexibles de maintien sont constitués de griffes (11) radiales disposées sur les parois du corps cylindrique (10), **caractérisé en ce que** les griffes sont inclinées en direction des fosses nasales (7), de façon à ne pas pouvoir être déplacées par le péristaltisme naturel du canal lacrymal entraînant larmes et corps étrangers vers l'intérieur.

2. Bouchon lacrymal selon la revendication 1, **se caractérisant par le fait que** les griffes (11) sont de longueur constante.

3. Bouchon lacrymal selon la revendication 1, **se caractérisant par le fait que** les griffes (11) sont de longueur variable.

4. Bouchon lacrymal selon la revendication 3, **se caractérisant par le fait que** les griffes (11) sont de longueur croissante ou décroissante.

5. Bouchon lacrymal selon l'une quelconque des revendications précédentes, **se caractérisant par le fait que** les griffes (11) sont disposées en hélice autour du corps (10).

6. Bouchon lacrymal selon l'une quelconque des revendications précédentes, **se caractérisant par le fait qu'**il comporte un ou plusieurs éléments tels que disques flexibles agencés pour assurer son étanchéité.

7. Bouchon lacrymal selon l'une quelconque des revendications précédentes, **se caractérisant par le fait que** les éléments flexibles sont déterminés pour présenter une élasticité suffisante pour s'enfoncer partiellement dans la paroi interne (12) du canalicule lacrymal (4, 5) en se redressant, de manière à assurer fermement le maintien en place du bouchon lacrymal.

8. Bouchon lacrymal selon l'une quelconque des revendications précédentes, **se caractérisant par le fait qu'**il est pourvu d'un conduit axial (13) permettant un passage réduit des larmes.

9. Bouchon lacrymal selon l'une quelconque des revendications précédentes, **se caractérisant par le fait que** le corps (10) est en forme de cône, de double cône ou de diabolo.

10. Bouchon lacrymal selon l'une quelconque des revendications précédentes, **se caractérisant par le fait qu'**il est réalisé en métal.

11. Bouchon lacrymal selon l'une quelconque des revendications 1 à 9, **se caractérisant par le fait qu'**il est réalisé en métal à mémoire de forme.

12. Bouchon lacrymal selon l'une quelconque des revendications précédentes, **se caractérisant par le fait qu'**il comporte un repère radio-opaque visible aux rayons X pour faciliter le repérage au cours de sa progression lors de sa mise en place.

13. Instrument pour la mise en place d'un bouchon lacrymal conforme aux revendications précédentes, **caractérisé en ce qu'**il est constitué d'un tube (15) à poussoir (16) agencé pour permettre de plaquer les griffes (11) contre la paroi externe de l'élément (10) et de les libérer une fois la prothèse en place.

14. Instrument pour la mise en place d'un bouchon lacrymal conforme aux revendications 1 à 12, **caractérisée en ce qu'**il est pourvu de machoires similaires à celles d'un porte-mine, ledit instrument étant agencé pour permettre également d'opérer le retrait de la prothèse.

## Claims

1. Lachrymal plugs primarily designed to allow the occlusion of the lachrymal ducts in order to cure a deficiency of the lachrymal glands by reducing or eliminating the flow of the tears towards the nasal cavity, comprising a roughly cylindrical body (10) on the outer sidewalls of which are flexible elements that can be applied against the walls of the aforesaid cylindrical body to allow the insertion of the lachrymal plug, and to straighten up when they are released in order to hold the aforementioned lachrymal plug in place, in which the plug is adapted to be inserted in the lachrymal duct (4, 5) and the flexible elements consisting of radial flexible elements (11) arranged on the walls of cylindrical body (10),
**characterised in that** the flexible elements are arranged inclined in the direction of nasal cavities (7) to prevent their removal by natural peristalsis of the lachrymal duct carrying tears and foreign bodies towards the inside.

2. Lachrymal plug according to claim 1, **characterised in that** flexible elements (11) are of constant length.

3. Lachrymal plug according to claim 1, **characterised in that** flexible elements (11) are of variable length.

4. Lachrymal plug according to claim 3, **characterised in that** flexible elements (11) are of increasing or decreasing length.

5. Lachrymal plug according to any of the preceding claims, **characterised in that** flexible elements (11) are in a helical arrangement around body (10).

6. Lachrymal plug according to any of the preceding claims, **characterised in that** it comprises one or more elements such as flexible discs arranged to ensure it remains tight.

7. Lachrymal plug according to any of the preceding claims, **characterised in that** the flexible elements are flexible enough to be inserted partially in inner wall (12) of lachrymal duct (4, 5) while straightening to ensure that the lachrymal plug is firmly held in place.

8. Lachrymal plug according to any of the preceding claims, **characterised in that** it has an axial duct (13) allowing reduced flow of tears.

9. Lachrymal plug according to any of the preceding claims, **characterised in that** body (10) has a cone, double cone or twinned wheel form.

10. Lachrymal plug according to any of the preceding claims, **characterised in that** it is made of metal.

11. Lachrymal plug according to any of claims 1 to 9, **characterised in that** it is made out of shape-memory metal.

12. Lachrymal plug according to any of the preceding claims, **characterised in that** it comprises a radio-opaque mark visible to x-rays which facilitates its positioning during its installation.

13. Instrument for installing a lachrymal plug complying with the preceding claims, **characterised in that** it consists of a tube (15) with push rod (16) arranged to allow the flexible elements (11) to be set against the outer wall of element (10) and to release them once the prothesis is in place.

14. Instrument for installing a lachrymal plug in compliance with claims 1 to 12, **characterised in that** it is equipped with jaws similar to those of a propelling pencil, the aforementioned instrument being arranged to also allow withdrawal of the prothesis.

## Patentansprüche

1. Tränengangverschluss, im wesentlichen zum Verschluss der Tränengänge bestimmt, um bei einer Fehlfunktion der Tränendrüsen Abhilfe durch Verringerung oder Unterbrechung des Tränenflusses in die Nasalhöhlen zu schaffen, bestehend aus einem annähernd zylindrischen Körper (10), auf dessen äußeren Seitenwänden flexible Elemente angeordnet sind, die gegen die Wand des genannten zylindrischen Körpers angelegt werden können, um das Einführen des Tränengangverschlusses zu ermöglichen, und die sich, wenn sie freigegeben werden, wieder aufrichten können, so dass der eingesetzte Tränengangverschluss festgehalten wird, wobei der Verschluss so gestaltet ist, dass er in Längsrichtung eines Tränenganges (4,5) eingesetzt wird und wobei die flexiblen Elemente aus radialen, auf den Wänden des zylindrischen Körpers (10) angeordneten Stäbchen (11) bestehen, **gekennzeichnet dadurch, dass** die Stäbchen in Richtung der Nasalhöhlen (7) geneigt sind, so dass sie nicht durch die natürliche Peristaltik des Tränenganges, die Tränen und Fremdkörper nach innen befördert, verschoben werden können.

2. Tränengangverschluss gemäss Anspruch 1, **gekennzeichnet dadurch, dass** die Stäbchen (11) eine konstante Länge besitzen.

3. Tränengangverschluss gemäss Anspruch 1, **gekennzeichnet dadurch, dass** die Stäbchen (11) eine variable Länge besitzen.

4. Tränengangverschluss gemäss Anspruch 3, **gekennzeichnet dadurch, dass** die Stäbchen (11) eine zunehmende oder abnehmende Länge besitzen.

5. Tränengangverschluss gemäss einem der vorstehenden Ansprüche, **gekennzeichnet dadurch, dass** die Stäbchen (11) schraubenförmig um den Körper (10) angeordnet sind.

6. Tränengangverschluss gemäss einem der vorstehenden Ansprüche, **gekennzeichnet dadurch, dass** er ein oder mehrere Elemente, wie zum Beispiel flexible Scheiben besitzt, die so angeordnet sind, dass sie ihn abdichten.

7. Tränengangverschluss gemäss einem der vorstehenden Ansprüche, **gekennzeichnet dadurch, dass** die flexiblen Elemente so ausgelegt sind, dass sie eine ausreichende Elastizität besitzen, um sich beim Wiederaufrichten teilweise in die Innenwand (12) des Tränenkanals (4, 5) einzudrücken, um den Tränengangverschluss auf diese Weise sicher an seinem Platz zu halten.

8. Tränengangverschluss gemäss einem der vorstehenden Ansprüche, **gekennzeichnet dadurch, dass** er einen axialen Kanal (13) besitzt, der einen verringerten Durchfluss von Tränenflüssigkeit erlaubt.

9. Tränengangverschluss gemäss einem der vorstehenden Ansprüche, **gekennzeichnet dadurch, dass** der Körper (10) die Form eines Kegels, eines an der Spitze oder eines an der Basis zusammengesetzten Doppelkegels besitzt.

10. Tränengangverschluss gemäss einem der vorstehenden Ansprüche, **gekennzeichnet dadurch, dass** er aus Metall ist.

11. Tränengangverschluss gemäss einem der Ansprüche 1 bis 9, **gekennzeichnet dadurch, dass** er aus einem metallischen Werkstoff mit Formgedächtnis ist.

12. Tränengangverschluss gemäss einem der vorstehenden Ansprüche, **gekennzeichnet dadurch, dass** er eine für Röntgenstrahlen undurchsichtige Markierung besitzt, die im Röntgenbild sichtbar ist, um seine Verfolgung beim Einführen zu erleichtern.

13. Instrument zur Einführung eines Tränengangverschlusses gemäss den vorstehenden Ansprüchen, **gekennzeichnet dadurch, dass** es aus einem Rohr (15) mit Vorschieber (16) besteht, der so gestaltet ist, dass er die Stäbchen (11) an die äußere Wand des Elements (10) andrückt und diese loslässt, sobald die Prothese eingesetzt ist.

14. Instrument zum Einsetzen eines Tränengangverschlusses gemäss den Ansprüchen 1 bis 12, **gekennzeichnet dadurch, dass** es Klemmbacken besitzt, ähnlich wie ein Minenhalter, und dass dieses Instrument so gestaltet ist, dass es auch zum Herausziehen der Prothese dienen kann.
